# EUROPÄISCHE PATENTSCHRIFT

(11) **EP 0 727 078 B1**
(45) Veröffentlichungstag und Bekanntmachung des Hinweises auf die Patenterteilung: **12.05.1999**
(21) Anmeldenummer: 95931971.6
(22) Anmeldetag: 01.09.1995
(51) Int. Cl.: G07F 7/06, G07F 7/00, B65G 1/137

(54) **AUTOMATISCHES GETRÄNKETERMINAL**
AUTOMATIC TERMINAL FOR DISTRIBUTING CRATES OF DRINKS
TERMINAL DE DISTRIBUTION AUTOMATIQUE DE CAISSES DE BOISSONS

(30) Priorität: 07.09.1994 DE 4431870
(43) Veröffentlichungstag der Anmeldung: 21.08.1996
(73) Patentinhaber: Heumann, Cordula, D-90455 Nürnberg (DE); Heumann, Friedrich, D-90455 Nürnberg (DE)
(72) Erfinder: Heumann, Cordula, D-90455 Nürnberg (DE); Heumann, Friedrich, D-90455 Nürnberg (DE)
(74) Vertreter: Weber, Joachim, Dr.
(86) Internationale Anmeldenummer: EP9503449
(87) Internationale Veröffentlichungsnummer: WO9607993

(56) Entgegenhaltungen:
- EP-A- 0 010 686
- EP-A- 0 590 646
- EP-A- 0 591 845
- WO-A-87/01102
- DE-U- 9 403 105
- FR-A- 2 540 080
- FR-A- 2 694 487
- GB-A- 903 228
- US-A- 3 343 638
- US-A- 3 719 287
- US-A- 4 253 573
- US-A- 5 186 281

## Beschreibung

Die Erfindung bezieht sich auf ein Getränketerminal zur automatischen Ausgabe von Getränkekästen.

Die aus dem Stand der Technik bekannten Verkaufsstätten für Getränkekästen sind im wesentlichen in Form sogenannter SB-Märkte ausgebildet, bei welchen der Kunde innerhalb eines Marktes Getränkekästen zu einer Kasse transportieren muß, um sie nach dem Zahlvorgang zu dem Kundenfahrzeug zu transportieren. In ähnlicher Weise erfolgt die Rückgabe des Leergutes, dieses wird meist separat an einer Kasse abgegeben. Nachteilig hierbei ist, daß der gesamte Kaufvorgang tür den Kunden aufwendig und beschwerlich ist und daß ein derartiger Getränkemarkt einen großen Flächenbedarf aufweist, da auch ausreichende Verkehrswege für die Kunden vorhanden sein müssen. Weiterhin ist ein hoher Personalaufwand erforderlich, um die Getränkekästen nachzufüllen, um den Zahlvorgang durchzuführen und um die Kunden zu betreuen.

Ein weiterer Nachteil bekannter SB-Märkte liegt darin, daß diese den Ladenschlußzeiten unterliegen, so daß der Kunde nur während einiger Stunden des Tages seine Einkäufe tätigen kann.

Die DE-PS 33 01 905 betrifft einen selbstkassierenden Warenautomaten, bei welchem einzelne Flaschen, welche auf geneigte Vollgutspeicher aufgesetzt wurden, wahlweise über einen Querförderer einer Warenausgabe zugeleitet werden können. Neben der Warenausgabe ist eine Leergutannahme vorgesehen, durch welche unter Verwendung des Querförderers Leergut auf geneigte Leergutspeicher-Bahnen aufbringbar ist. Dieser Warenautomat ist in der Lage, einzelne Getränkeflaschen abzugeben bzw. aufzunehmen.

Es ist weder ein vollautomatischer Betriebsablauf vorgesehen noch ist es möglich, mittels dieser Konstruktion größere Einheiten, beispielsweise Getränkekästen zu handhaben oder gar diese von Paletten zu entladen bzw. wieder auf Paletten aufzustapeln.

Der Zeitschriftenartikel "Transport- Förder- und Lagertechnik, 1984, Heft 4, Seiten 9 bis 12" beschreibt ein Verteilerzentrum für Bücher, bei welchem ankommende Paletten in ein Hochregallager eingesetzt werden. Die Paletten werden aus dem Hochregallager entweder im Betrieb verteilt oder in Teilmengen abgepackt und in ein Zwischenlager überführt. Diesem ist ein Kommissionierlager nachgeordnet, in welchem einzelne Bücher zur nachfolgenden Verpackung und Kommissionierung zwischengelagert werden. Das gesamte Lagersystem ist rechnergesteuert.

Den nächst kommenden Stand der Technik zeigen die EP-A2-590 646 und die DE-OS 42 32 833. Bei diesen vorbekannten automatischen Getränketerminals erfolgt die Zwischenlagerung der Getränkekästen in einem Verkaufslager, welches entweder durch geneigte Rollenbahnen oder durch Karussell-Lager gebildet ist. Bei geneigten Rollenbahnen kann das Problem auftreten, daß einzelne Getränkekästen durch Scherben, Verunreinigungen oder ähnliches oder durch Verkanten oder Verrutschen der Kästen selbst blockiert werden. Ein Karussell-Lager kann, abhängig von den jeweiligen Einsatzbedingungen und der Speicherkapazität des Verkaufslagers eine zu lange Zugriffszeit aufweisen.

Der Erfindung liegt die Aufgabe zugrunde, ein Getränketerminal zur automatischen Ausgabe von Getränkekästen zu schaffen, welches bei vollautomatischer Betriebsweise ohne jegliches Personal dem Kunden bei betriebssicherer und schneller Arbeitsweise eine Vielzahl von Getränkesorten anbieten kann, und welches sich durch sehr kurze Zugriffszeiten auf das Verkaufslager auszeichnet.

Erfindungsgemäß wird die Aufgabe durch die Merkmale des Hauptanspruchs gelöst, die Unteransprüche weisen vorteilhafte Ausgestaltungen der Erfindung auf.

Das erfindungsgemäße Getränke-Terminal gestattet es somit, in automatisierter Weise Getränkegebinde an einen Kunden zu verkaufen, diese Getränkegebinde können entweder in Form von Getränkekästen oder in Form von Einweggebinden ausgestaltet sein. Bei Getränkekästen nehmen diese üblicherweise Mehrweg-Flaschen auf, welche zusammen mit den Getränkekästen gegen einen entsprechenden Pfand abgegeben bzw. gegen Pfandrückgabe zurückgenommen werden. Bei Einweggebinden erfolgt keine Rücknahme der Gebinde, diese werden vielmehr separat entsorgt. In Abhängigkeit von der jeweiligen Ausgestaltung der Getränkekästen bzw. der Gebinde kann somit eine Anpassung des Getränketerminals erfolgen. Der Begriff Getränkekästen umfaßt im Sinne der vorliegenden Erfindung beides, nämlich Getränkekästen mit Mehrwegflaschen sowie Gebinde mit Einwegflaschen, Dosen, Kunststoffverpackungen oder ähnlichem.

Gemäß einer bevorzugten Weiterbildung der Erfindung sind bei der Anpassung des Getränketerminals an Mehrweg-Getränkekästen und Mehrweg-Flaschen eine Leergut-Annahme-Stelle sowie entsprechende Leergutspeicher und Bepalettiereinrichtungen vorgesehen. Diese Einrichtungsgegenstände können entfallen, wenn Einweggebinde verwendet werden.

Nachfolgend wird ein Ausführungsbeispiel der Erfindung zugrundegelegt, bei welchem Getränkekästen mit Mehrweg-Flaschen zum Einsatz kommen. Ein derartiges Getränketerminal weist somit in bevorzugter Weise folgende Baugruppen auf: Ein Lieferterminal für die Anlieferung von mit Getränkekästen beladenen Paletten, zumindest ein Hochregal-Fahrzeug, welches zur Bevorratung der Paletten in einem Hochregal dient, eine Vereinzelungseinrichtung zum Vereinzeln der Getränkekästen, zumindest ein Verkaufslager, in welchem die einzelnen Getränkekästen bevorratet sind, zumindest ein Kunden-Kastenfahrzeug bzw. eine Kunden-Fördereinrichtung zur Entnahme der Kästen aus dem Verkaufslager und zur Übergabe der Kästen an eine Getränkeausgabestelle, welche mit einer Kundenkonsole versehen ist. Erfindungsgemäß ist weiterhin vorgesehen, daß die Vereinzelungseinrichtung und die Bepalettiereinrichtung zumindest einen horizontal angeordneten Linearförderer umfassen, welcher einer Lagerfläche des Verkaufslagers zugeordnet ist und mit Greifmitteln zum Greifen und Heben/Senken von einzelnen Getränkekästen versehen ist.

Das erfindungsgemäße Getränketerminal zeichnet sich durch eine Reihe erheblicher Vorteile aus. Der wesentliche Vorteil liegt darin, daß das Getränketerminal gänzlich ohne jedes Personal arbeitet, so daß die Betriebskosten erheblich reduziert werden können. Weiterhin ist nur ein sehr geringer Platzbedarf erforderlich, da keine durch die Kunden zu befahrenden Verkehrswege erforderlich sind. Durch die vollautomatische Steuerung ergeben sich sehr kurze Zugriffszeiten, so daß der Kunde die gewünschten Getränkekästen wesentlich schneller in Empfang nehmen kann. Da der gesamte Ablauf des Getränketerminales vollautomatisch erfolgt, ist auch die gesamte Abwicklung, Nachfüllung und Lagerhaltung optimierbar.

Bei dem erfindungsgemäßen Getränketerminal handelt es sich um eine gänzlich neue technische Anlage, welche bisher keinerlei Vorbild hatte. Dies resultiert insbesondere aus dem hohen Gewicht der Getränkekästen, aus der Bruchempfindlichkeit der Flaschen und aus dem Problem einer geeigneten Handhabung der Getränkekästen. Zugegebenermaßen sind auf dem Gebiet der Lagertechnik unterschiedlichste Teil-Komponenten vorbekannt, diese wurden jedoch, wie auch die zitierten Entgegenhaltungen zeigen, noch nie in einer funktionsfähigen Gesamtanlage kombiniert. Zusätzlich zu den Anforderungen an die Lager- und Regeltechnik kommen bei einem Getränketerminal weitere Probleme hinzu, die erfindungsgemäß erstmals gelöst wurden. So ist das Käuferverhalten zu berücksichtigen. Für den Käufer scheiden längere Wartezeiten aus, er ist nicht bereit, minutenlang auf die Anlieferung der Waren zu warten. Demgemäß muß das Getränketerminal so konstruiert sein, daß sich sehr kurze Zugriffszeiten ergeben. Weiterhin ist der Kunde nicht bereit, auf andere Produkte auszuweichen, wenn das gewünschte Produkt nicht verfügbar ist. Aus diesem Grunde muß ein Getränketerminal über eine ausreichende Lagerhaltung verfügen, welche automatisch so nachfüllbar ist, daß auch in Stoßzeiten die Kundenbelieferung sichergestellt ist. Ein weiteres Problem ergibt sich aus der Leergutrücknahme. Da diese aus Umweltschutzgründen zwingend erforderlich ist, muß das Getränketerminal zwei komplette Lager-Bereiche umfassen, nämlich ein Lager für volle Getränkebehälter und ein Leergutlager. Beide müssen so verknüpft sein, daß sich ein funktionssicherer Betriebsablauf ergibt.

Die Kundenkonsole ist bevorzugterweise zur bargeldlosen Zahlungsabwicklung mit einem Kartenleser versehen, so daß der Kunde seine EC-Karte zur Abwicklung des Kaufvorganges verwenden kann. Durch die einzutippende Geheimzahl ist ein hohes Maß an Betriebssicherheit gewährleistet. Da weiterhin derartige EC-Karten nicht an jugendliche ausgegeben werden, ist auch sichergestellt, daß keine Jugendlichen oder Kinder alkoholische Getränke kaufen können. Um den Kunden auch die problemlose Rückgabe von Leergut zu ermöglichen, ist der Kundenkonsole bevorzugterweise eine Leergut-Annahmestelle zugeordnet. Diese steht mit einem Leergut-Speicher in Verbindung, welchem eine Bepalettierungeinrichtung nachgeordnet sein kann, um die Leergutkästen auf Paletten zu stapeln und diese automatisch transportieren zu können.

Um den gesamten Anliefervorgang zu automatisieren, kann es günstig sein, wenn im Bereich des Lieferterminales eine Leseeinrichtung zur Bestimmung der angelieferten, palettierten Getränkekästen vorgesehen ist. Es ist somit möglich, den Getränketerminal per LKW mit Vollpaletten zu versorgen, welche jeweils sortenrein mit gleichen Getränkekästen beladen sind. Der LKW-Fahrer entlädt, beispielsweise mittels eines Staplers eine Vollpalette und fährt sie zum Lieferterminal. Dort wird mittels eines Barcode-Lesers die Getränkesorte bestimmt. Falls sich hierbei Probleme ergeben, kann der Fahrer auch mittels eines Handscanners per Hand eine Identifikation der Getränkesorte vornehmen. Von dem Anlieferterminal aus wird die beladene Palette nach einem chaotischen Lagersystem in dem Hochregallager abgestellt. In ähnlicher Weise kann aus dem Hochregallager eine leere Palette oder eine mit Leergutkästen beladene Palette entnommen oder an das Lieferterminal übergeben werden, damit der Fahrer das Leergut oder die leere Palette abtransportieren kann.

Der beschriebene Liefervorgang kann an unterschiedlich ausgestalteten Lieferterminales vorgenommen werden. So ist es möglich, das Lieferterminal mit einem Lastenaufzug auszurüsten, so daß mehrere Betriebsebenen des Getränketerminales nutzbar sind.

Das Lieferterminal kann, gesteuert durch eine zentrale Recheneinheit auch so bedient werden, daß ohne Rückgabe von Leergutpaletten oder leeren Paletten lediglich eine Befüllung mit vollen oder leeren Paletten möglich ist, beispielsweise zum Anlaufen des Getränketerminales. Es können auch einzelne, auswählbare Paletten ausgeschleust werden, beispielsweise Leergutpaletten, Vollgutpaletten oder leere Paletten, beispielsweise um das Getränketerminal leer zu fahren oder um, beispielsweise bei Erreichen des Verfallsdatums bestimmte Getränkepaletten zu entnehmen.

Um den Anliefervorgang bzw. den Vorgang des Abtransportes von Leergutpaletten überwachen zu können, kann es günstig sein, wenn im Bereich des Lieferterminales ein Lieferscheindrucker vorgesehen ist, welcher dem Fahrer automatisch einen Lieferschein ausstellt.

Die zwischen dem Hochregallager und dem Verkaufslager vorgesehene Vereinzelungseinrichtung umfaßt in günstiger Weise eine Entpalettiervorrichtung sowie zumindest ein Vereinzelungsfahrzeug, welches einzelne Getränkekästen von der Entpalettiervorrichtung entnimmt und in das Verkaufslager überführt.

Gemäß der Erfindung werden die in der Vereinzelungseinrichtung einzelnen Getränkekästen über zwangsweise angetriebene Förderbahnen einem Verkaufslager zugeführt.

Durch die Verwendung des erfindungsgemäß vorgesehenen Linearförderers ist es möglich, die einzelnen Getränkekästen auf einer horizontalen Lagerfläche des Verkaufslagers abzustellen. Dieses Zwischenlagern in dem Verkaufslager kann nach einem chaotischen Lagersystem erfolgen, so daß die Gesamtfläche des Verkaufslagers optimal ausgenutzt werden kann. Da die Getränkekästen sowohl nebeneinander als auch übereinander abstellbar sind, ist es möglich, die einzelnen Getränkesorten passend zu dem jeweiligen Lagerbestand und den Durchsatzmengen abzustellen. Da der Linearförderer, welcher als Linearroboter ausgebildet ist, somit jeweils einen direkten Zugriff auf den gewünschten Getränkekasten hat, ergeben sich sehr kurze Zugriffszeiten. Weiterhin können die Zugriffswege optimiert werden, beispielsweise durch geeignete Deponierung häufig benötigter Getränkesorten an bewegungstechnisch günstigen Bereichen des Linearförderers.

In günstiger Weiterbildung der Erfindung ist vorgesehen, daß der Linearförderer eine horizontale Schiene umfaßt, welche auf Streben in einem Abstand zur Lagerfläche aufgesetzt ist. Durch die Hochsetzung der Schienen ergibt sich ein großes vertikales Speichervolumen. Da der Linearförderer die Verkaufslager-Fläche übergreift, kann die Fläche mit optimaler Ausnutzung mit Getränkekästen belegt werden, es sind keine Verkehrswege etc. freizuhalten.

Weiterhin ist es günstig, wenn auf der Schiene ein Wagen verfahrbar ist, welcher die Greifmittel trägt. An dem Wagen kann eine Quertraverse gelagert sein, welche sich quer zu der Schiene erstreckt und welche in ihrer Längsrichtung relativ zu dem Wagen verfahrbar sein kann. Hierdurch wird der seitliche Aktionsradius des Linearförderers erheblich gesteigert, so daß das Speichervolumen des Verkaufslagers erhöht werden kann. Es ist selbstverständlich auch möglich, die Quertraverse an zwei zueinander parallelen Schienen zu lagern.

Um die Palettierung bzw. Bepalettierung mittels des Linearförderers zu erleichtern und um die dem Kundenterminal zuzuleitenden Getränkekästen sicher verarbeiten zu können, ist es günstig, wenn der Linearförderer Förderbahnen umgreift, welche der Vereinzelungseinrichtung, der Kunden-Fördereinrichtung und/oder der Bepalettiereinrichtung zugeordnet sind. Der Linearförderer kann somit Getränkekästen direkt von diesen Förderbahnen entnehmen bzw. auf diese aufsetzen. Weiterhin kann der Linearförderer diese Getränkekästen entweder direkt von einer Förderbahn auf die andere übergeben oder in dem Verkaufslager abstellen bzw. aus diesem entnehmen.

Die Greifmittel sind bevorzugterweise so ausgebildet, daß sie vertikal auf den oberen Randbereich des Getränkekastens absenkbar und von diesem hochfahrbar sind. Die Greifmittel, welche mit Kupplungsmitteln zum lösbaren Greifen des Getränkekastens versehen sein können, können somit den Getränkekasten selektiv anheben bzw. abstellen, unabhängig von der Anzahl oder dem Füllzustand der in dem Getränkekasten befindlichen Flaschen. Somit können sowohl Getränkekästen mit gefüllten Flaschen als auch Getränkekästen mit Leergut oder leere Getränkekästen in gleicher Weise betriebssicher gehandhabt werden.

Mittels einer Steuer- und Recheneinheit, welche den Linearförderer steuert, ist es möglich, die jeweilige Abstellposition eines Getränkekastens zu speichern bzw. diesen Kasten wieder aufzufinden. Hierzu kann der Kasten mit geeigneten Datenträgern, beispielsweise Barcodes oder ähnlichem Versehen sein, um seinen Inhalt zu identifizieren. Weiterhin können Mittel vorgesehen sein, um Art und Größe des Getränkekastens zu unterscheiden, beispielsweise Bierkästen, Wasserkästen, teilbare Spezial-Kästen oder Kästen in Sondergrößen.

Das Kunden-Kastenfahrzeug kann zur Aufnahme mehrerer Getränkekästen ausgebildet sein, um die erforderlichen Fahrwege zu verringern.

Im folgenden wird die Erfindung anhand eines Ausführungsbeispieles in Verbindung mit der Zeichnung beschrieben. Dabei zeigt:
- Fig. 1: in schematischer Darstellung eine Draufsicht auf die Ausgestaltung eines erfindungsgemäßen Getränketerminales,
- Fig. 2: eine vereinfachte perspektivische Darstellung des Verkaufslagers mit dem Linearförderer,
- Fig. 3: eine Seitenansicht des Verkaufslagers mit dem Linearförderer,
- Fig. 4: ein Flußdiagramm, welches den Ablauf der Bedienungsvorgänge des Kunden an einer Kundenkonsole wiedergibt, und
- Fig. 5: eine Ansicht einer Kundenkonsole.

In den Zeichnungen sind gleiche Teile jeweils mit gleichen Bezugsziffern bezeichnet.

Bei dem nachfolgend beschriebenen Ausführungsbeispiel wird ein Getränketerminal beschrieben, welches für Mehrweg-Flaschen bzw. Mehrweg-Kästen verwendet wird. Die Erfindung ist jedoch nicht auf diese Ausgestaltungsform beschränkt, vielmehr ist es im Rahmen der Erfindung ebensogut möglich, auf die Leergut-Annahme-Stelle und die zugeordneten Einrichtungen, insbesondere den Leergutspeicher und die Bepalettiereinrichtung zu verzichten.

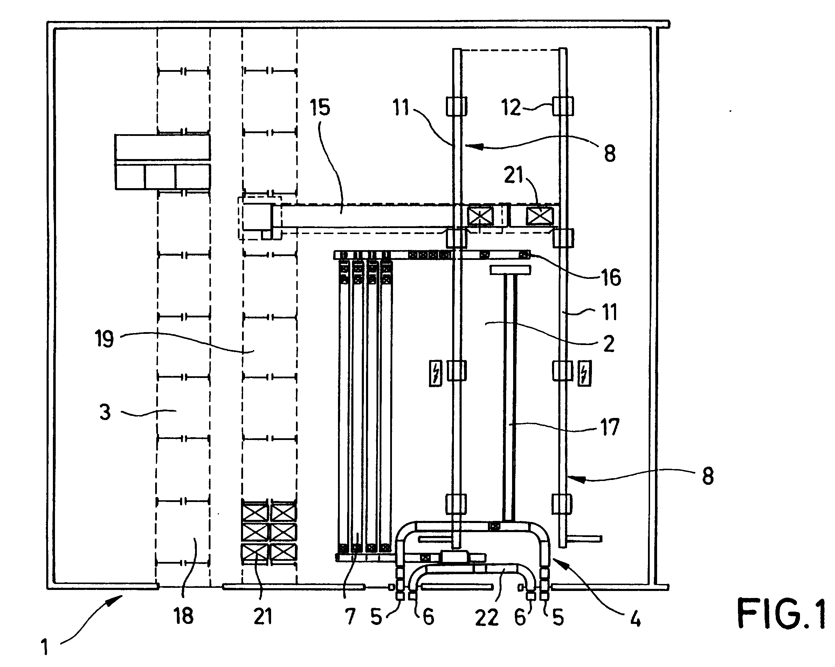

Die Fig. 1 zeigt in der Draufsicht den Aufbau eines Ausführungsbeispieles eines erfindungsgemäßen Getränketerminales. Dieses umfaßt ein Lieferterminal 1, welches beispielsweise auch in einer anderen Ebene angeordnet sein kann. Somit können Paletten zu einem LKW befördert bzw. von diesem entnommen werden. Das Lieferterminal 1 umfaßt zwei zueinander parallele Rollbahnen 18, 19, eine Rollbahn zur Anlieferung von Paletten, die andere Rollbahn zur Abgabe von Paletten an ein nicht dargestelltes Lieferfahrzeug. Beide Rollbahnen 18 und 19 sind von einem Hochregal-Fahrzeug anfahrbar, welches auf Schienen innerhalb eines Hochregales 3 verfahrbar ist.

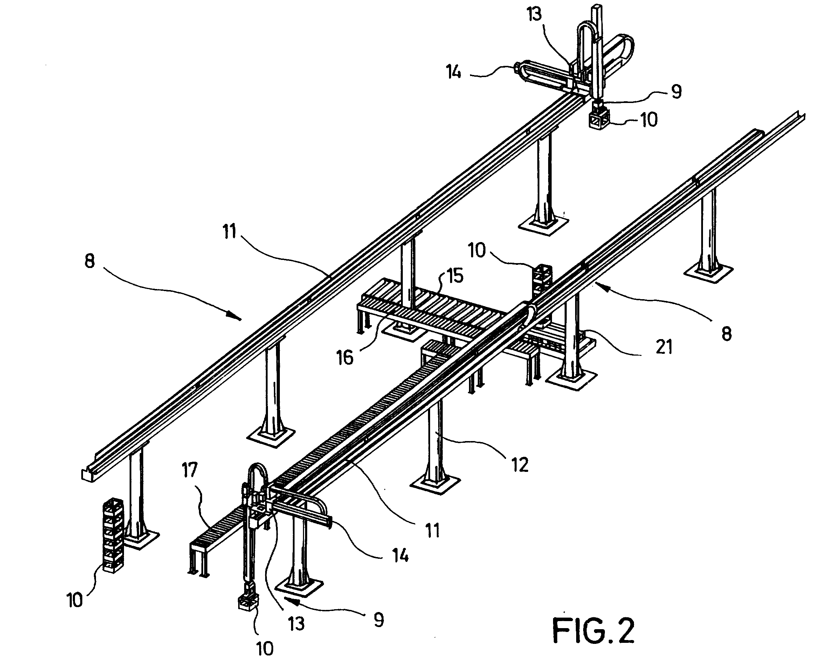

Die Palettierung bzw. Bepalettierung der Paletten erfolgt im Bereich zweier zueinander paralleler Linearförderer 8. Diese weisen Schienen 11 auf, welche auf Streben 12 aufgesetzt sind und sich linear erstrecken. Auf der Schiene 11 läuft, wie in Fig. 2 dargestellt, ein Wagen 13, welcher eine Quertraverse 14 trägt. An der Quertraverse 14 sind Greifmittel 9 vorgesehen, um selektiv einen Getränkekasten 10 zu greifen.

Mittels des Linearförderers 8 ist es somit möglich, einen Getränkekasten zu greifen, diesen im angehobenen Zustand zu verfahren und an einer vorbestimmten Stelle abzusetzen. Das Absetzen der Getränkekästen kann nebeneinander oder in einem Stapel übereinander erfolgen.

Die Bepalettierung von Paletten 21, welche nur schematisch dargestellt sind, erfolgt folgendermaßen:

Die mit Getränkekästen beladenen Paletten (sh. auch Fig. 2) werden mittels einer Förderbahn 15 von dem Hochregallager 3 in den Arbeitsbereich des Linearförderers 8 überführt. Dort sind sie für die Greifmittel 9 zugänglich. Nunmehr werden einzelne Kästen nacheinander von der Palette 21 entnommen und auf den Boden des Verkaufslagers 2 abgestellt.

Die leeren Paletten 21 können über die Förderbahn 15 wieder in das Hochregallager 3 zurücktransportiert werden. Es ist jedoch auch möglich, Leergut, welches sich zwischenzeitlich in einem Leergutspeicher 7 angesammelt hat und welches über eine Förderbahn 16 in den Arbeitsbereich des Linearförderers 8 überführt wurde, auf eine leere Palette 21 zu stapeln.

Der Leergutspeicher 7 umfaßt mehrere zueinander parallele Förderbahnen. Hierdurch können die einzelnen unterschiedlichen Leergutkästen separat zwischengespeichert werden.

Die Fig. 1 zeigt weiterhin, daß zwischen den Linearförderern 8 eine weitere Förderbahn 17 angeordnet ist, welche mit einer Kundenfördereinrichtung 4 verbunden ist. Die Kunden-fördereinrichtung 4 endet in einer Getränkeausgabestelle 5 einer Kundenkonsole. Somit können die auf das Förderband oder die Förderbahn 17 abgestellten Getränkekästen über die Kundenfördereinrichtung 4 der Kundenausgabestelle 5 zugeleitet werden.

Die Förderbahnen 15, 16, 17 können als Förderbänder, als Rollenförderwege oder in ähnlicher Weise ausgebildet sein. Gleiches gilt für die Kundenfördereinrichtung 4.

Im Bereich der Kundenkonsole ist weiterhin eine Leergutannahmestelle 6 vorgesehen, welche über eine Leergutförderbahn 22 mit dem Leergutspeicher 7 verbunden ist. Das von einem Kunden abgegebene Leergut kann somit nach erfolgter Leergutkennung und Prüfung in den Leergutspeicher 7 überführt werden.

Die Fig. 2 zeigt in perspektivischer Ansicht den Aufbau des erfindungsgemäßen Verkaufslagers 2 und zeigt dabei insbesondere den Arbeitsbereich der Linearförderer 8 sowie diesen zugeordneten Förderbahnen 15, 16 und 17.

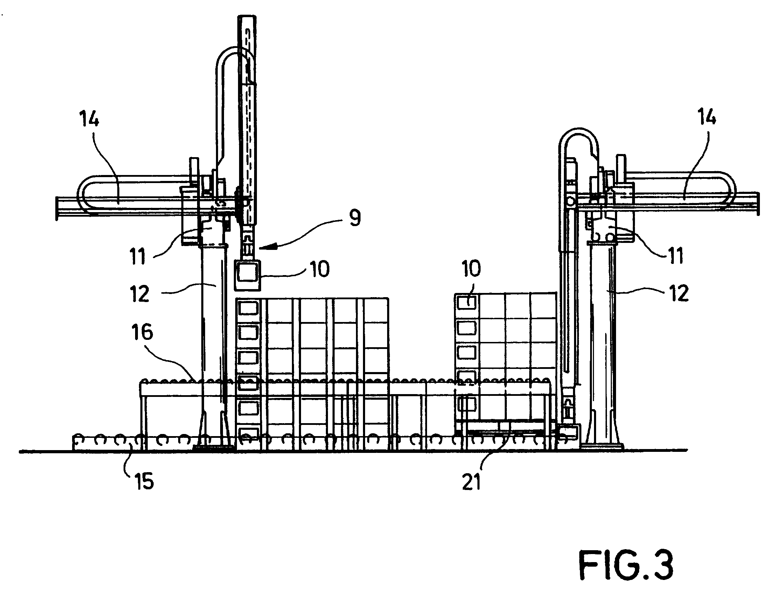

Aus Fig. 3 ist eine Seitenansicht des in Fig. 2 gezeigten Verkaufslagers ersichtlich.

In dem Verkaufslager (Pufferlager) können beispielsweise jeweils 5 Kästen einer Getränkesorte übereinander gestapelt werden. Hierdurch ist beispielsweise eine Kapazität von ca. 2.200 Kästen erreichbar. Das Schlicht-Muster, nach welchem die einzelnen Kästen abgestellt werden, kann dabei in Abhängigkeit von der jeweiligen Abverkaufsmenge vorgegeben werden. Das Schlicht-Muster kann sich selbstverständlich, wie bei einem chaotischen Lagersystem üblich, ständig ändern, um das Verkaufslager den jeweiligen Einsatzbedingungen anzupassen.

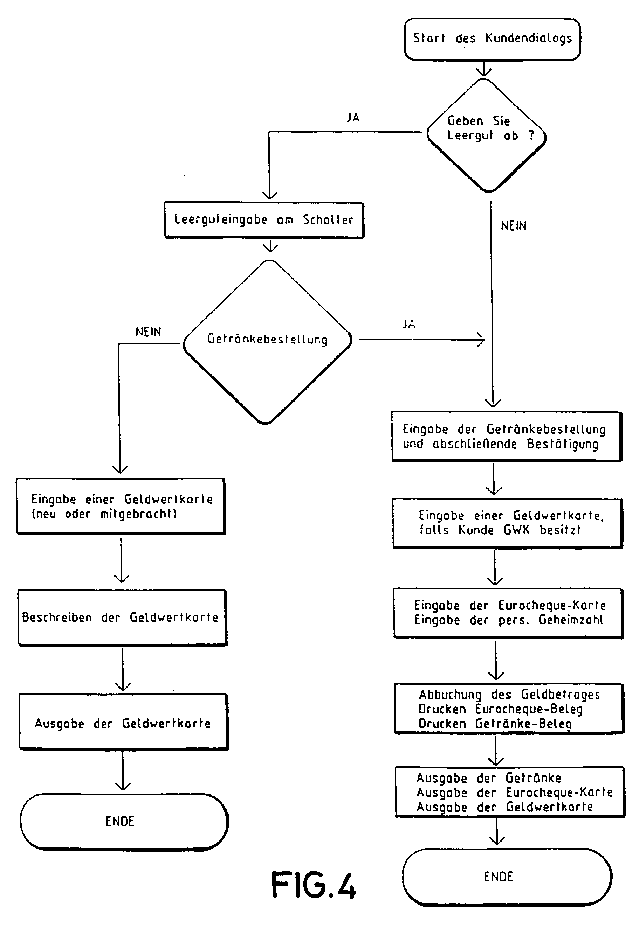

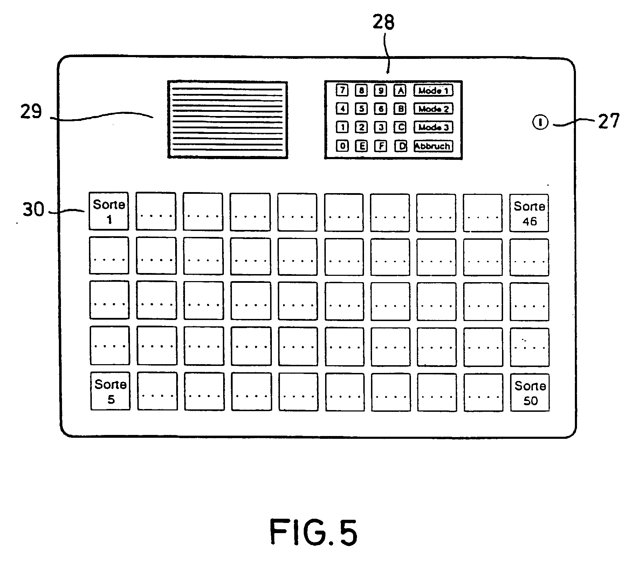

Die Fig. 5 zeigt eine Ansicht des Teiles der Kundenkonsole 7. Zunächst weist diese einen Schlüsselschalter 27 auf, um durch das Bedienungspersonal manuelle Funktionen durchführen zu können. Die Fig. 5 zeigt weder die Eingabe für eine EC-Karte noch die Ausgabe einer Geldwertkarte zur Abrechnung des zurückgegebenen Leergutes. An der Bedienerkonsole sind Funktionstasten 28 vorgesehen, um beispielsweise die EC-Geheimzahl einzugeben und um die Betriebsarten auszuwählen. An einem Display 29 wird zunächst der Kunde gefragt, ob er, wie in Fig. 4 gezeigt, Leergut abgeben möchte. Sofern er dies bejaht, wird das Leergut in die Leergut-Annahmestelle 6 eingegeben und dort automatisch geprüft. Es wird ermittelt, ob die Leergutkästen annahmefähig sind, weiterhin wird der Füllzustand mit leeren Flaschen festgestellt. Erforderlichenfalls wird dem Kunden dann das nicht akzeptierbare Leergut zurückgegeben. Der Wert des Leergutes kann bei einem nachfolgenden Einkauf sofort verrechnet werden, es ist auch möglich, dem Kunden einen Leergut-Wertkarte auszugeben, auf welcher der anzurechnende oder auszuzahlende DM-Betrag gespeichert ist. Sofern der Kunde den Betriebsmodus "Getränkebestellung" wählt, kann er über Getränkeauswahltasten 30 die gewünschte Getränkesorte bestellen. Falls der Kunde eine Geldwertkarte besitzt, kann er diese zusätzlich zur Verrechnung eingeben. Nachdem die gewünschten Getränkekästen aus dem Verkaufslager 2 entnommen und der Getränke-Ausgabestelle 5 zugeführt wurden, wird der erforderliche Geldbetrag automatisch von der EC-Karte abgebucht, der Kunde erhält durch einen nicht dargestellten Drucker einen Beleg ausgedruckt. Aus dieser Beschreibung ergibt sich, daß der gesamte Funktionsablauf automatisch erfolgt und daß keinerlei Bargeldtransfer stattfindet. Hierdurch erhöht sich die Betriebssicherheit. Die Gefahr von Überfällen oder Einbrüchen wird, da kein Bargeld vorhanden ist, beseitigt.

Mittels einer zentralen Rechnereinheit werden die einzelnen Funktionsabläufe der Getränketerminales überwacht, es wird u.a. der Füllzustand des Hochregallagers und des Verkaufslagers ermittelt, um rechtzeitig Nachbestellungen vornehmen zu können. All dies erfolgt automatisch, ebenso wie die wegoptimierte Steuerung sämtlicher Fahrzeuge, um eine möglichst kurze Zugriffszeit zu realisieren. Es versteht sich, daß Notfunktionen vorgesehen sind, um beispielsweise das Getränketerminal leerzufahren oder um gezielt einzelne Funktionsaufläufe beeinflussen oder überbrücken zu können.

## Patentansprüche

1. Getränketerminal zur automatischen Ausgabe von Getränkekästen, mit einem Lieferterminal (1) für mit Getränkekästen beladene Paletten, zumindest einem Hochregal (3), einer Vereinzelungseinrichtung einer Bepalettiereinrichtung, zumindest einem Verkaufslager (2), zumindest einer Kunden-Fördereinrichtung (4), zumindest einer mit einer Kundenkonsole versehenen Getränkeausgabestelle (5), dadurch gekennzeichnet, daß die Vereinzelungseinrichtung und die Bepalettiereinrichtung zumindest einen horizontal angeordneten Linearförderer (8) umfassen, welcher einer Lagerfläche des Verkaufslagers (2) zugeordnet ist und mit Greifmitteln (9) zum Greifen und Heben/Senken von einzelnen Getränkekästen (10) versehen ist.

2. Getränketerminal nach Anspruch 1, gekennzeichnet durch eine der Kundenkonsole (5) zugeordnete Leergut-Annahme-Stelle (6), einen mittels der Kunden-Fördereinrichtung (4) beschickbaren Leergutspeicher (7), und eine dem Leergutspeicher (7) nachgeordnete Bepalettiereinrichtung, welche mit dem Hochregal (3) verbunden ist.

3. Getränketerminal nach Anspruch 2, dadurch gekennzeichnet, daß der Linearförderer (8) eine horizontale Schiene (11) umfaßt, welche auf Streben (12) in einem Abstand zur Lagerfläche aufgesetzt ist.

4. Getränketerminal nach Anspruch 3, dadurch gekennzeichnet, daß auf der Schiene (11) ein Wagen (13) verfahrbar ist, welcher die Greifmittel (8) trägt.

5. Getränketerminal nach Anspruch 4, dadurch gekennzeichnet, daß an dem Wagen (13) eine Quertraverse (14) gelagert ist, welche sich quer zu der Schiene (11) erstreckt und die Greifmittel (9) trägt.

6. Getränketerminal nach Anspruch 5, dadurch gekennzeichnet, daß die Quertraverse (14) in ihrer Längsrichtung relativ zu dem Wagen (13) verfahrbar ist.

7. Getränketerminal nach einem der Ansprüche 3 bis 6, dadurch gekennzeichnet, daß der Linearförderer (8) Förderbahnen (15, 16, 17) übergreift, welche der Vereinzelungseinrichtung, der Kunden-Fördereinrichtung (4) und/oder der Bepalettiereinrichtung zugeordnet sind.

8. Getränketerminal nach einem der Ansprüche 3 bis 7, dadurch gekennzeichnet, daß die Greifmittel (9) vertikal auf den oberen Randbereich des Getränkekastens (10) absenkbar und von diesem hochfahrbar sind.

9. Getränketerminal nach Anspruch 8, dadurch gekennzeichnet, daß die Greifmittel (9) mit Kupplungsmitteln zum lösbaren Greifen des Getränkekastens (10) versehen sind.

10. Getränketerminal nach eine der Ansprüche 1 bis 9, dadurch gekennzeichnet, daß der Linearförderer (8) einen mit einer Steuer- und Recheneinheit verbundenen Antrieb aufweist.

11. Getränketerminal nach Anspruch 10, dadurch gekennzeichnet, daß die Steuer- und Recheneinheit zur Ablage und zum Aufnehmen von Getränkekästen (10) ausgebildet ist, welche nebeneinander und/oder übereinander angeordnet sind.

## Claims

1. A drinks terminal for automatically dispensing drinks boxes, having a delivery terminal (1) for pallets charged with drinks boxes, at least one high storage unit (3), a separating device, a palleting device, at least one retail storage area (2), at least one customer conveyor (4), at least one drinks dispensing position (5) provided with a customer console,
**characterised in that** the separating device and the palleting device comprise at least one horizontally disposed linear conveyor (8), which is associated with a floor space of the retail storage area (2) and is provided with gripping means (9) for gripping and raising/lowering individual drinks boxes.

2. A drinks terminal according to Claim 1,
**characterised by** an empties acceptance point (6) associated with the customer console (5), an empties storage area (7) chargeable by means of the customer conveyor (4) and a palleting device provided behind the empties storage area (7), which is connected to the high storage unit (3).

3. A drinks terminal according to Claim 2,
**characterised in that** the linear conveyor (8) comprises a horizontal rail (11) which is placed onto stanchions (12) at a distance from the floor area.

4. A drinks terminal according to Claim 3,
**characterised in that** a carriage (13), which bears the gripping means (8), is movable on the rail (11).

5. A drinks terminal according to Claim 4,
**characterised in that** mounted on the carriage (13) is a crosshead (14), which extends transversally to the rail (11) and bears the gripping means (9).

6. A drinks terminal according to Claim 5,
**characterised in that** the crosshead (14) is movable in its longitudinal direction in relation to the carriage (13).

7. A drinks terminal according to one of Claims 3 to 6,
**characterised in that** the linear conveyor (8) engages over conveyor tracks (15, 16, 17), which are associated with the separating device, the customer conveyor (4) and/or the palleting device.

8. A drinks terminal according to one of Claims 3 to 7,
**characterised in that** the gripping means (9) can be lowered onto the upper edge region of the drinks box (10) and can be raised therefrom.

9. A drinks terminal according to Claim 8,
**characterised in that** the gripping means (9) are provided with coupling means for the releasable gripping of the drinks box (10).

10. A drinks terminal according to one of Claims 1 to 9,
**characterised in that** the linear conveyor (8) comprises a drive connected to a control and arithmetic-logic unit.

11. A drinks terminal according to Claim 10,
**characterised in that** the control and arithmetic-logic unit is constructed to deposit and accept drinks boxes (10), which are disposed next to one another and/or above one another.

## Revendications

1. Terminal de distribution de caisses de boissons pour la distribution automatique de caisses de boissons, avec un terminal de livraison (1) pour les palettes chargées de caisses de boissons, au moins un rayonnage surélevé (3), un arrangement d'individualisation, un arrangement de palettisation, au moins une réserve de vente (2), au moins un convoyeur menant aux clients (4), au moins un poste de distribution de boissons (5) muni d'un pupitre de commande pouvant être manipulé par les clients, caractérisé en ce que l'arrangement d'individualisation et l'arrangement de palettisation comprennent au moins un convoyeur linéaire (8) disposé horizontalement, auquel est associée une surface d'entreposage de la réserve de vente (2) et muni de moyens de préhension (9) pour saisir et soulever/abaisser les caisses de boissons individuelles (10).

2. Terminal de distribution de caisses de boissons selon la revendication 1, caractérisé par un poste de réception de récipients vides (6) associé au pupitre de commande pouvant être manipulé par les clients (5), un entrepôt pour les récipients vides (7) pouvant être chargé par le convoyeur menant aux clients (4) et un arrangement de palettisation placé à la suite de l'entrepôt pour les récipients vides (7), ledit arrangement étant relié au rayonnage surélevé (3).

3. Terminal de distribution de caisses de boissons selon la revendication 2, caractérisé en ce que le convoyeur linéaire (8) comprend un rail horizontal (11), qui est posé sur des étais à une certaine distance de la surface d'entreposage.

4. Terminal de distribution de caisses de boissons selon la revendication 3, caractérisé en ce qu'un chariot (13) peut se déplacer sur le rail (11), ledit chariot supportant les moyens de préhension (8).

5. Terminal de distribution de caisses de boissons selon la revendication 4, caractérisé en ce que sur le chariot (13) est posée une traverse (14) qui s'étend perpendiculairement au rail (11) et qui supporte les moyens de préhension (9).

6. Terminal de distribution de caisses de boissons selon la revendication 5, caractérisé en ce que la traverse (14) peut se déplacer dans le sens de sa longueur par rapport au chariot (13).

7. Terminal de distribution de caisses de boissons selon les revendications 3 à 6, caractérisé en ce que le convoyeur linéaire (8) recouvre les lignes de convoyage (15, 16, 17), qui sont associées à l'arrangement d'individualisation, au convoyeur menant aux clients (4) et/ou à l'arrangement de palettisation.

8. Terminal de distribution de caisses de boissons selon l'une des revendications 3 à 7, caractérisé en ce que les moyens de préhension (9) peuvent être abaissés verticalement jusqu'au niveau de la périphérie supérieure de la caisse de boissons (10) et levés à partir de cette périphérie.

9. Terminal de distribution de caisses de boissons selon la revendication 8, caractérisé en ce que les moyens de préhension (9) sont munis de moyens d'accouplement pour établir une prise amovible de la caisse de boissons (10).

10. Terminal de distribution de caisses de boissons selon l'une des revendications 1 à 9, caractérisé en ce que le convoyeur linéaire (8) présente un entraînement relié à une unité de commande et de comptage.

11. Terminal de distribution de caisses de boissons selon la revendication 10, caractérisé en ce que l'unité de commande et de comptage est construite pour l'empilement et la réception de caisses de boissons (10) qui sont disposées les unes à côté des autres et/ou les unes sur les autres.
